# EUROPÄISCHE PATENTANMELDUNG

(11) **EP 2 821 475 A1**
(43) Veröffentlichungstag der Anmeldung: **07.01.2015**
(21) Anmeldenummer: 14175365.7
(22) Anmeldetag: 02.07.2014
(51) Int. Cl.: C12M 1/107, C12M 1/00, C12M 1/02, C12M 1/34

(54) **Biogas-Erzeugung aus Biomasse**

(30) Priorität: 02.07.2013 DE 102013106953
(71) Anmelder: Lutz, Peter, 81925 Unterföhring (DE)
(72) Erfinder: Lutz, Peter, 81925 Unterföhring (DE)
(74) Vertreter: Alber, Norbert

(57) **Zusammenfassung**

Bei einer Biogasanlage nach dem Feststoff-Gärverfahren wird durch nur eine einzige Perkolat-Verteilvorrichtung sichergestellt, dass ausschließlich zum Beispiel hygienisiertes Perkolat in den einzelnen Fermentern (1) ausgebracht wird, unabhängig davon ob das im unteren Bereich der Fermenter (1) gesammelte Perkolat hygienisiert war oder nicht. Zu diesem Zweck wird das Perkolat vor dem Einbringen in den Perkolat-Ausbringungsbehälter (3b) in einer Behandlungseinrichtung (7) hygienisiert, sofern es sich um nicht bereits im Fermenter (1) hygienisiertes Perkolat handelt.

## Beschreibung

### I. Anwendungsgebiet

Die Erfindung betrifft die sogenannte Biogas-Erzeugung aus Biomasse.

### II. Technischer Hintergrund

Heute wird Biomasse, beispielsweise Mais in frischer Form oder als Silage, auf zwei grundsätzlich unterschiedliche Arten anaerob vergoren:
- einerseits im Nassgär-Verfahren, bei dem die Biomasse in Form einer pumpfähigen Schlempe vorliegt,
- andererseits im hier vorliegenden Feststoff-Verfahren, bei der die Biomasse, nur durch das Animpfen mit bereits vergorenem Material, in die Fermenter eingebracht wird.

Bei diesem Feststoff-Verfahren, dem so genannten Trockenfermentationsverfahren, sammelt sich Sickerflüssigkeit, das so genannte Perkolat, am Boden der Fermenter, welches aus der in der Biomasse enthaltenen Flüssigkeit besteht, die durch das Eigengewicht der Biomasse heraus gepresst wird, und die mit Fortschritt des Umsetzungsprozesses zusätzlich biologisch aktive Gär-Stoffe enthält.

Dabei ist es üblich, dieses Perkolat aus dem Bodenbereich der Fermenter abzupumpen und wieder auf der Oberseite der Biomasse im Fermenter zu versprühen.

Dadurch werden die biologisch aktiven Stoffe im Perkolat gleichmäßig über das gesamte Volumen der Biomasse im Fermenter verteilt, indem das Perkolat flächig von oben nach unten durch die Biomasse hindurchsickert. Gleichzeitig kann eine Temperaturregelung im Fermenter mittels des zirkulierenden Perkolates erreicht werden, indem dieses vor der Rückführung nach Bedarf beheizt wird.

Dabei unterscheidet sich sowohl die Menge als auch eventuell die Inhaltsstoffe des einem Fermenter zuzuführenden Perkolates abhängig davon, in welchem Zustand der Umsetzungsprozesse sich befindet, welche Art von ursprünglicher Biomasse er enthält und aufgrund von weiteren Faktoren.

Bislang wurden die aus dem Fermenter entnommenen Gärreste in der Regel bei der nachfolgenden, aeroben, Kompostierung hygienisiert.

Inzwischen wird die in einem Fermenter umgesetzte Biomasse vor der Entnahme aus dem Fermenter dort hygienisiert.

Hygienisieren bedeutet in diesem Fall gemäß Bioabfall-Verordnung, dass nicht sämtliche in der Biomasse enthaltenen Keime abgetötet werden sollen, sondern es sollen zum einen in der Biomasse enthaltene Samen deaktiviert werden, damit diese später nicht in Form von Unkraut wieder aufgehen können, und es sollen vor allem bestimmte Koli-Bakterien dabei abgetötet werden. Die den anaeroben Gärprozess bewirkenden bioaktiven Gärstoffe sollen dagegen gerade nicht abgetötet werden, und dies ist auch durch entsprechende Temperaturführung möglich.

Sobald dieser Hygienisierungsprozess einsetzt, ist es selbstverständlich kontraproduktiv, wenn dann noch Perkolat in den Fermenter zugeführt wird, welches selbst noch nicht hygienisiert ist.

Die EP 2 202 292 A1 beschreibt eine gattungsgemäße Biogas-Anlage, bei der zwei getrennte Perkolat-Behälter vorhanden sind, und jeweils eine Gruppe von Fermentern mit einem der beiden Perkolat-Behälter verbunden ist und aus diesem versorgt wird. Ein Austausch von Perkolat zwischen den beiden Perkolat-Behältern erfolgt nicht.

Dies erfordert eine aufwändige Verleitung für das Perkolat, da jeder einzelne Fermenter sowohl auf der Zuführungsseite als auch auf der Abführungsseite mit jedem der beiden Perkolat-Behälter verbunden können sein muss und in der Zuleitung zu und in der Ableitung von jedem Fermenter entsprechende Ventile usw. vorgesehen werden müssen.

Des Weiteren wird durch diese Vorgehensweise die Steuerung der Anlage wesentlich komplizierter und auch fehleranfälliger, denn wenn aus Versehen beispielsweise ein Fermenter gefüllt mit nachwachsenden Rohstoffen mit Perkolat besprüht wird, welches aus einem Fermenter mit biologischem Hausmüll kommt, ist unter Umständen dieser Gärrets aus diesem Fermenter nur noch Sondermüll.

### III. Darstellung der Erfindung

### a) Technische Aufgabe

Es ist daher die Aufgabe gemäß der Erfindung, eine Biogasanlage sowie ein Verfahren für ihren Betrieb zur Verfügung zu stellen, welche mit geringem Investitionsaufwand herzustellen und einfach zu betreiben ist.

### b) Lösung der Aufgabe

Diese Aufgabe wird durch die Merkmale der Ansprüche 1 und 6 gelöst. Vorteilhafte Ausführungsformen ergeben sich aus den Unteransprüchen. Hinsichtlich des **Verfahrens** wird die erfindungsgemäße Aufgabe dadurch gelöst, dass ausschließlich hygienisiertes Perkolat in den Fermentern auf die Biomasse ausgebracht wird.

Die Ausbringung eines falschen Perkolats in einen der Fermenter, wie sie bei wahlweiser Ausbringung unterschiedlicher Arten von Perkolat auf den einzelnen Fermentern erfolgen kann, ist dadurch zuverlässig vermieden.

In der Regel liegen zwei Arten von Perkolat, nämlich hygienisiertes und nicht hygienisiertes, Perkolat vor. Häufig werden diese in getrennten diesbezüglichen Perkolat-Behältern gesammelt, wovon der Perkolat-Behälter für das hygienisierte Perkolat als Ausbringungsbehälter bezeichnet wird.

Die Zirkulation von Perkolat durch die Biogas-Anlage, also durch die einzelnen Fermenter, kann theoretisch jedoch auch ohne Perkolat-Behälter realisiert werden mit Hilfe von ausreichend groß dimensionierten Zirkulationsleitungen oder ähnlichem. In der Praxis hat sich das Zwischenspeichern in Perkolat-Behältern jedoch bewährt, da hierdurch auch ein unregelmäßiger Anfall von Perkolat bewältigt werden kann.

Nicht hygienisiertes Perkolat aus dem entsprechenden Perkolat-Behälter kann in den Ausbringungsbehälter für hygienisiertes Perkolat verbracht werden, und wird dabei oder spätestens im Ausbringungsbehälter hygienisiert. Zum Hygienisieren wird in der Regel Wärme verwendet, alternativ stehen auch chemische und/oder physikalische Behandlungsverfahren zur Verfügung.

Das in den Fermentern anfallende Perkolat kann entweder grundsätzlich zunächst dem ersten Perkolat-Behälter für nicht hygienisiertes Perkolat zugeführt werden, selbst wenn aus dem einen oder anderen Fermenter bereits hygienisiertes Perkolat austritt, da die Biomasse in dem entsprechenden Fermenter bereits den Hygienisierungsschritt durchlaufen hat.

Diese Variante stellt sicher, dass nicht aus Versehen unhygienisiertes Perkolat in den Ausbringungsbehälter gelangen kann.

Eine andere Variante besteht darin, dass das in den einzelnen Fermentern anfallende Perkolat abhängig von seinen Eigenschaften, beispielsweise ob es sich um hygienisiertes oder nicht hygienisiertes Perkolat handelt, den entsprechenden Perkolat-Behältern zugeführt wird, dann natürlich über getrennte Leitungen.

Diese Variante vermeidet es, dass bereits hygienisiertes Perkolat nochmals hygienisiert werden muss.

Hinsichtlich der konstruktiven Ausgestaltung der **Biogas-Anlage** wird die erfindungsgemäße Aufgabe dadurch gelöst, dass die Anlage zwar mindestens zwei Perkolat-Behälter umfasst, jedoch die Perkolat-Verteilvorrichtung nur mit einem der beiden Perkolat-Behälter, dem Ausbringungs-Behälter, verbunden ist, welcher ausschließlich hygienisiertes Perkolat enthält. Zusätzlich ist in der Verbindungsleitung zwischen den beiden Perkolat-Behältern eine Behandlungseinheit für das Perkolat vorhanden, um das Perkolat in den für den Ausbringungs-Behälter notwendigen Zustand zu bringen.

In baulicher Hinsicht ergibt dies den Vorteil eines geringeren Aufwandes, denn es muss nur eine Hauptleitung für die Perkolatverteilung von dem einen Ausbringungsbehälter zu allen Fermentern verlegt werden.

Die Perkolat-Entnahmevorrichtung aus de Fermentern kann dagegen durchaus zwei Entnahmestränge umfassen, wobei jeder Fermenter mit jedem der beiden Entnahmestränge verbunden ist, am anderen Ende jeder der beiden Entnahmestränge jedoch nur mit einem der beiden Perkolat-Behälter verbunden ist.

Da die Fermenter individuell hinsichtlich des Perkolates abgepumpt werden müssen, ist jedem Fermenter eine eigene, separat ansteuerbare Pumpe zugeordnet.

Die einzelnen Fermenter müssen hinsichtlich Zeitpunkt und Menge individuell mit Perkolat beschickt werden können, weshalb vorzugsweise von der Hauptleitung (gegebenenfalls mit Durchflussmessung) der Perkolat-Verteilvorrichtung Abzweigleitungen zu jedem Fermenter führen, in welchen mindestens je ein Sperrventil angeordnet ist.

### c) Ausführungsbeispiele

Ausführungsformen gemäß der Erfindung sind im Folgenden beispielhaft näher beschrieben. Es zeigen:
- Fig. **1:**: eine Biogas-Anlage mit nur einem Perkolat-Entnahmestrang,
- Fig. **2:**: eine Biogas-Anlage mit zwei Perkolat-Entnahmesträngen.

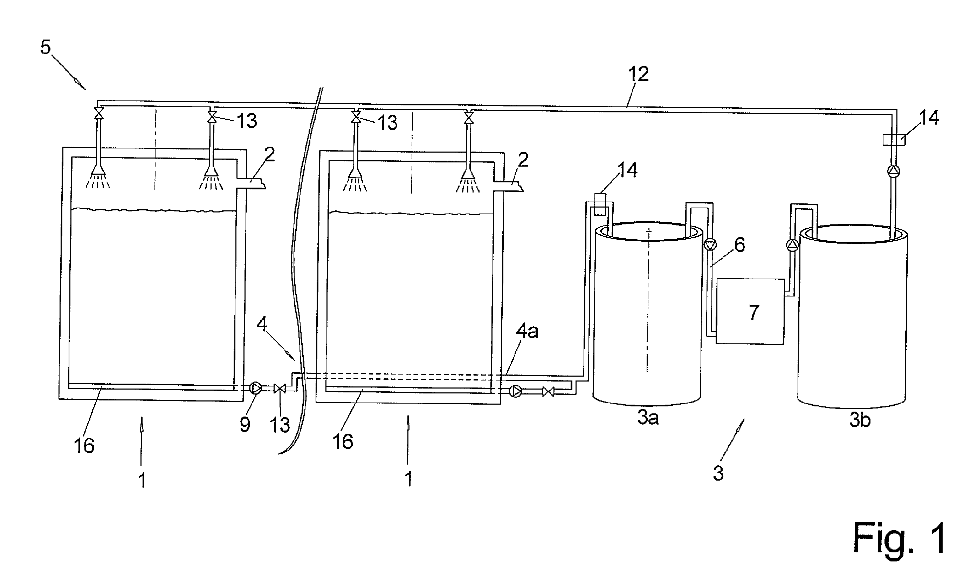

**Figur 1** zeigt eine Biogas-Anlage, die im Wesentlichen aus - in der Realität meist wesentlich mehr als den hier dargestellten nur zwei - Fermentern **1,** dargestellt in der Front-Ansicht und im geöffneten Zustand, besteht, die im Betrieb zum Großteil mit Biomasse gefüllt sind, die in den luftdicht und gasdicht verschlossenen Fermentern **1** unter Luftabschluss, also anearob, vergären soll und dabei zum Teil in ein brennbares Gasgemisch umgesetzt wird, welches aus den einzelnen Fermentern **1** über eine Biogas-Entnahmeöffnung **2** abgezogen und einem nicht dargestellten Verbraucher zugeführt wird.

Bei dem vorliegenden Verfahren wird die Biomasse in die Fermenter **1** eingebracht, die meist wie eine Fertiggarage gefertigt sind, also nur eine offene Frontseite aufweisen, durch die Biomasse eingebracht und entnommen werden kann, und die durch eine entsprechende dichte Klappe gasdicht verschlossen werden kann. Allerdings sind die Dimensionen der Fermenter demgegenüber wesentlich größer.

Die in den Fermentern **1** eingebrachte Biomasse enthält Feuchtigkeit, die allein schon durch das Eigengewicht der Biomasse im Fermenter und der Einlagerungshöhe durch Zusammenpressen der Biomasse ausgepresst wird und sich am Boden der Fermenter **1** sammelt als Sickerflüssigkeit, das sog. Perkolat, welches auch die für den Gärprozess notwendigen bioaktiven Substanzen enthält, die beispielsweise zu Beginn des Umsetzungsprozesses und auch danach der Biomasse im Fermenter zugeführt werden, indem ein Perkolat, das diese Stoffe enthält, auf der Oberseite der Biomasse im Fermenter **1** versprüht wird mit Hilfe einer Perkolat-Verteilvorrichtung **5.**

Im Gegenzug muss das Perkolat im unteren Bereich der Fermenter **1** über eine Perkolat-Entnahmevorrichtung **4** aus den einzelnen Fermentern **1** entfernt werden, um ausreichend Perkolat für das Besprühen von oben zur Verfügung zu haben.

Häufig ist die Bodenplatte des Fermenters mit einem Gefälle zur vorderen, verschließbaren Stirnseite hin ausgestattet, und in diesem vorderen Bereich ist eine über die gesamte Breite des Fermenters 1 verlaufende Sammelrinne 16 im Boden des Fermenters eingelassen, sodass sich darin das Perkolat sammelt.

Wie **Figur 1** zeigt, ist die Sammelrinne **16** jedes der Fermenter **1** über eine Stichleitung mit einem zu einem ersten Perkolatbehälter **3**a führenden Entnahmestrang 4a verbunden.

In jeder Stichleitung ist zumindest ein Sperrventil **13** und/oder vorzugsweise auch eine separat ansteuerbare Pumpe **9** angeordnet, sodass das Abpumpen aus jedem der einzelnen Fermenter **1** unabhängig voneinander erfolgen kann, beispielsweise abhängig vom Füllstand an Perkolat in der Sammelrinne **16.**

Durch entsprechende Sperrventile **13** in den Stichleitungen von den einzelnen Fermentern kann sichergestellt werden, dass ein Fluss des Perkolats in dem Entnahmestrang **4**a nur in den Perkolatbehälter **3**a hinein erfolgt und nicht unbeabsichtigterweise zurück in einen der übrigen Fermenter **1.**

Mit dem ersten Perkolatbehälters **3**a ist ein zweiter Perkolat-Behälter **3**b verbunden. Mittels einer Verbindungsleitung **6** und entsprechender ein oder mehrerer Pumpen kann Perkolat aus dem ersten Perkolat-Behälter 3a in den zweiten Perkolatbehälter **3**b gepumpt werden.

Dabei ist in der Verbindungsleitung **6** eine Behandlungseinheit **7** für Perkolat eingesetzt, in der das hindurchfließende Perkolat so behandelt wird, dass es diejenigen Eigenschaften besitzt, die das Perkolat für das anschließende Ausbringen des Perkolats aus dem zweiten Perkolat-Behälter **3**b, dem Ausbringungsbehälter, besitzen muss, um auf der Biomasse in den Fermentern **1** verteilt zu werden.

Beispielsweise wird in der Behandlungseinheit **7** das hindurchströmende Perkolat hygienisiert.

Auch eine Zumischung notwendiger weiterer Bestandteile im richtigen Verhältnis kann in der Behandlungseinheit **7** durchgeführt werden.

Im Gegensatz zu bisherigen Lösungen wird nun Perkolat nicht wahlweise aus einem der beiden Perkolat-Behälter, sondern immer nur aus dem gleichen Ausbringungsbehälter **3**b verwendet, um es auf der Biomasse der einzelnen Fermenter **1** zu verteilen, um dadurch einerseits eine gute Verteilung der Gärstoffe im Inneren der Biomasse durch das Hindurchsickern nach unten zu gewährleisten und andererseits bei Bedarf auch eine Temperatursteuerung in der Biomasse durchzuführen.

Zu diesem Zweck führen von der Hauptleitung **12** (gegebenenfalls mit Durchflussmessung) der Perkolat-Verteilvorrichtung **5** in jeden Fermenter **1** ein oder mehrere Stichleitungen, die separat über Sperrventile **13** absperrbar sind, indem immer nur eines der Sperrventile **13** geöffnet ist.

In der Regel werden die einzelnen Fermenter aus Gründen der gleichmäßigen Biogas-Erzeugung zeitlich zueinander versetzt befüllt und der Vergärprozess begonnen, sodass der über mehrere Wochen laufende Gärprozess in den einzelnen Fermentern leicht zeitversetzt abläuft.

Im Rahmen des Umsetzungsprozesses der Biomasse ist es in der Regel erwünscht, dass die noch im Fermenter **1** verbliebene Biomasse - die ja in ihrem Volumen deutlich reduziert ist - hygienisiert ist, um sie anschließend als Dünger verwenden zu können, bzw. zu Qualitäts-Kompost umwandeln zu können oder ähnliches, ohne dass hierbei z.B. aktive Samen in der Biomasse diese Verwendung der Biomasse-Reste behindern.

Die Hygienisierung erfolgt in der Regel dadurch, dass im Inneren des Fermenters **1** die enthaltene Biomasse über einen bestimmten Zeitraum eine bestimmte Mindesttemperatur besitzt, wodurch die im Fokus stehenden Keime abgetötet werden. Dieses Temperaturniveau kann durch den Umsetzungsprozess selbst erreicht werden oder auch durch Zuheizung, da die Fermenter **1** ohnehin zumindest im Boden eine nicht dargestellte Heizvorrichtung besitzen, da eine Zuheizung unter anderem zu Beginn des Umsetzungsprozesses notwendig ist, um den Gärprozess in Gang zu setzen trotz der anfänglichen Zusetzung der richtigen bioaktiven Gärstoffe.

Sobald die Biomasse in einem der Fermenter **1** diese Hygienisierung bereits durchlaufen hat, ist auch das in seinem unteren Bereich austretende bzw. sich in der Sammelrinne **16** sammelnde Perkolat hygienisiert.

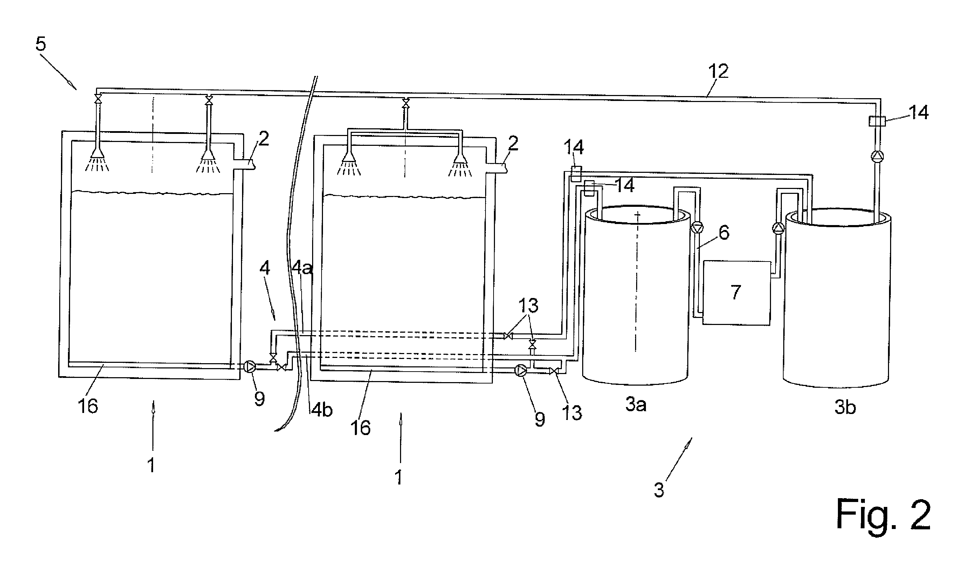

Falls der Unterschied zwischen den beiden Perkolat-Behältern **3**a und **3**b lediglich die Hygienisierung ist, also sich im Perkolat-Behälter **3**a nicht hygienisiertes Perkolat befindet und im Perkolat-Behälter **3**b ausschließlich bereits hygienisiertes Perkolat, kann - wie in **Figur 2** dargestellt - ein solcher bereits die Hygienisierung durchlaufener Fermenter **1** umgestellt werden auf Abführung seines Perkolats direkt in den hygienisierten Perkolat-Behälter **3**b:

In **Figur 2** umfasst die Perkolat-Entnahmevorrichtung **4** deshalb zwei parallele Entnahmestränge **4**a, b, von denen der eine wie bei Figur **1** im ersten Perkolat-Behälter **3**a und der zweite im zweiten Perkolat-Behälter **3**b mündet.

Jeder der Fermenter **1** ist über entsprechende Stichleitungen mit jeweils beiden Entnahmesträngen **4**a, b verbunden, und durch Sperrventile **13** in beiden Stichleitungen kann für jeden Fermenter **1** separat und zu jedem beliebigen Zeitpunkt umgeschaltet werden, wohin das Perkolat abgepumpt wird, also in den ersten Perkolat-Behälter **3**a oder in den zweiten Perkolat-Behälter **3**b.

### BEZUGSZEICHENLISTE

- **1**: Fermenter
- **2**: Biogas-Entnahmeöffnung
- **3**: Perkolat-Speicher
- **3**a, b: Perkolat-Behälter
- **4**: Perkolat-Entnahmevorrichtung
- **5**: Perkolat-Verteilvorrichtung
- **6**: Verbindungsleitung
- **7**: Behandlungseinheit
- **8**: Perkolat-Regelungseinrichtung
- **9**: Pumpe
- **10**: Zirkulationsrichtung
- **11**: Füllstandssensor
- **12**: Hauptleitung
- **13**: Sperrventil
- **14**: Durchflussmesser
- **15**: Rückschlagventil
- **16**: Sammelrinne

## Patentansprüche

1. Verfahren zur Erzeugung von Biogas aus nicht-pumpbarer Biomasse mit mehreren gas- und flüssigkeitsdicht verschließbaren Fermentern **(1)** für die Biomasse wobei
- das in den Fermentern **(1)** anfallende Perkolat gesammelt wird und
- in den einzelnen Fermentern **(1)** gesteuert wieder ausgebracht wird,
**dadurch gekennzeichnet, dass**
ausschließlich hygienisiertes Perkolat in den einzelnen Fermentern **(1)** ausgebracht wird.

2. Verfahren nach Anspruch **1,**
**dadurch gekennzeichnet, dass**
hygienisiertes und nicht hygienisiertes Perkolat in getrennten Perkolat-Behältern **(3**a,b) gesammelt wird, wovon der Perkolat -Behälter (3b) für hygienisiertes Perkolat der Ausbringungs-Behälter (**3**b) ist.

3. Verfahren nach einem der vorhergehenden Verfahrensansprüche,
**dadurch gekennzeichnet, dass**
das in den Ausbringungs-Behälter (**3**b) eingebrachte Perkolat vor oder spätestens im Ausbringungs-Behälter (3b) hygienisiert wird.

4. Verfahren nach einem der vorhergehenden Verfahrensansprüche,
**dadurch gekennzeichnet, dass**
das Hygienisieren mittels Wärme und/oder chemischer und/oder physikalischer Behandlung erfolgt.

5. Verfahren nach einem der vorhergehenden Verfahrensansprüche,
**dadurch gekennzeichnet, dass**
das in den Fermentern (**1**) anfallende Perkolat abhängig von seinen Eigenschaften wahlweise einem der beiden Perkolat-Behälter (**3**a,b), wieder zugeführt wird.

6. Biogasanlage zur Methanisierung von nicht-pumpbarer Biomasse, mit
- mehreren gas- und flüssigkeitsdicht verschließbaren Fermentern **(1)** für die Biomasse,
- einer Biogas-Entnahmeöffnung **(2)** in jedem Fermenter **(1),**
- einem Perkolat-Speicher **(3),**
- einer Perkolat-Entnahmevorrichtung **(4)** die aus jedem Fermenter **(1)** das dort anfallende Perkolat zu dem Perkolat-Speicher **(3)** befördert,
- einer Perkolat-Verteilvorrichtung **(5),** die das Perkolat aus dem Perkolat-Speicher **(3)** in jeden der Fermenter **(1)** ausbringen kann,
**dadurch gekennzeichnet, dass**
- der Perkolat-Speicher **(3)** mindestens zwei Perkolat-Behälter (**3**a, b) umfasst, die über eine Verbindungsleitung **(6)** miteinander verbunden sind,
- in der Verbindungsleitung **(6)** eine Behandlungseinheit (7) für das hindurchströmende Perkolat angeordnet ist,
- die Perkolat-Verteilvorrichtung **(5)** nur mit dem einen der beiden Perkolat-Behälter, dem Ausbringungs-Behälter (**3**b), verbunden ist.

7. Biogasanlage nach Anspruch **6,**
**dadurch gekennzeichnet, dass**
die Behandlungs-Einheit **(7)** eine Hygienisierungseinheit ist.

8. Biogasanlage nach einem der vorhergehenden Vorrichtungsansprüche,
**dadurch gekennzeichnet, dass**
die Perkolat-Entnahmevorrichtung **(4)** einen ersten Entnahmestrang (**4**a) und einen zweiten Entnahmestrang **4**b) umfasst, die einerseits jeweils mit jedem der Fermenter **(1)** verbunden sind und andererseits mit je einem der beiden Perkolat-Behälter (**3**a, b) verbunden sind.

9. Biogasanlage nach einem der vorhergehenden Vorrichtungsansprüche,
**dadurch gekennzeichnet, dass**
von den beiden Perkolat -Behältern (3a, b) in der Aufsicht betrachtet der eine konzentrisch innerhalb des anderen angeordnet ist.

10. Biogasanlage nach einem der vorhergehenden Vorrichtungsansprüche,
**dadurch gekennzeichnet, dass**
die Perkolat-Entnahmevorrichtung **(4)** jedem Fermenter **(1)** zugeordnet wenigstens eine separat steuerbare Pumpe **(9)** umfasst.

11. Biogasanlage nach einem der vorhergehenden Vorrichtungsansprüche,
**dadurch gekennzeichnet, dass**
die Perkolat-Verteilvorrichtung **(5)** eine Hauptleitung **(12)** umfasst, die gegebenenfalls einen Durchflussmesser **(15)** aufweist, und von der Abzweigleitungen **(13)** zu jedem Fermenter **(1)** führen, in welchen ein Sperrventil **(14)** angeordnet ist.
